# EUROPÄISCHE PATENTSCHRIFT

(11) **EP 1 728 470 B1**
(45) Veröffentlichungstag und Bekanntmachung des Hinweises auf die Patenterteilung: **03.12.2008**
(21) Anmeldenummer: 05012092.2
(22) Anmeldetag: 04.06.2005
(51) Int. Cl.: A61B 5/15, A61B 5/00, G01D 7/12

(54) **Analysehandgerät mit akustischer Ausgabe von Messergebnissen**
Portable device for analysis with acoustic output of measurement results
Appareil portable d'analyse avec emission acoustique des résultats de mesure

(43) Veröffentlichungstag der Anmeldung: 06.12.2006
(73) Patentinhaber: Roche Diagnostics GmbH, 68305 Mannheim (DE); F. Hoffmann-La Roche AG, 4070 Basel (CH)
(72) Erfinder: Kintzig, Hans, 67311 Tiefenthal (DE); Thilges, Jean, 69190 Walldorf (DE)
(74) Vertreter: Pfeifer, Hans-Peter

(56) Entgegenhaltungen:
- EP-A- 1 172 651
- WO-A-03/017832
- DE-A1- 4 334 273
- DE-A1- 19 902 972
- PATENT ABSTRACTS OF JAPAN Bd. 1997, Nr. 08, 29. August 1997 (1997-08-29) & JP 09 094231 A (KOKURITSU SHINTAI SHOGAISHA REHABILITATION CENTER SOUCHIYOU), 8. April 1997 (1997-04-08)

## Beschreibung

Die Erfindung betrifft ein Analysehandgerät zur Untersuchung einer medizinisch bedeutsamen Probe, insbesondere zur Ermittlung der Blutglucosekonzentration.

Aus der EP 1 172 651 A1 ist ein Analysehandgerät mit einem Display bekannt, das in eine externe Sprachausgabeeinheit gesteckt werden kann, so daß Kontakte geschlossen und Meßergebnisse verbal über die Sprachausgabeeinheit ausgegeben werden können. Für sehbehinderte Benutzer, die auf einem Display angezeigte Meßergebnisse nicht oder nur mit großer Mühe erkennen können, bringt das bekannte Analysesystem bestehend aus einem Analysehandgerät mit einer optischen Anzeigeeinrichtung und einer externen Sprachausgabeeinheit eine große Erleichterung.

Nachteilig an dem bekannten Analysesystem ist jedoch, daß die externe Sprachausgabeeinheit relativ groß, teuer und unhandlich ist. Deshalb ist es für Diabetiker, die ihren Blutzuckerspiegel mehrmals täglich messen müssen, mühsam, die externe Sprachausgabeeinheit mitzuführen. Dadurch wird der Benutzerkomfort des bekannten Systems eingeschränkt.

Aufgabe der Erfindung ist es deshalb, einen Weg aufzuzeigen, wie für sehbehinderte Diabetiker der Benutzerkomfort eines Analysesystems, das eine akustische Ausgabe von Meßergebnissen ermöglicht, gesteigert werden kann.

Diese Aufgabe wird erfindungsgemäß gelöst durch ein Analysehandgerät zur Untersuchung einer medizinisch bedeutsamen Probe, insbesondere zur Ermittlung der Blutglucosekonzentration von Diabetikern, umfassend eine Meßeinrichtung zum Messen der Konzentration eines Analyten in der Probe, eine Ausgabeeinrichtung zum Ausgeben von Meßergebnissen, die mittels der Meßeinrichtung ermittelt wurden, wobei die Ausgabeeinrichtung sowohl eine akustische Signalausgabeeinrichtung zur Ausgabe der Meßergebnisse durch nonverbale akustische Signale als auch eine drahtlose Schnittstelle zur Kommunikation mit einer externen Sprachausgabeeinheit umfaßt, über die Meßergebnisse verbal ausgegeben werden können, die Ausgabeeinrichtung einen Akustikmodus, in dem Meßergebnisse mittels der akustischen Signalausgabeeinrichtung ausgegeben werden, und einen Sprachausgabemodus, in dem Meßergebnisse über die Schnittstelle ausgegeben werden, hat, und ein Übergang der Ausgabeeinrichtung aus dem Akustikmodus in den Sprachausgabemodus durch Empfang eines von der Sprachausgabeeinheit ausgesandten Signals ausgelöst wird.

Die Aufgabe wird ferner gelöst durch ein Analysesystem umfassend ein solches Analysehandgerät und eine Sprachausgabeeinheit, die eine drahtlose Schnittstelle zur Kommunikation mit dem Analysehandgerät aufweist.

Durch die Kombination einer akustischen Signalausgabeeinrichtung zur Ausgabe der Meßergebnisse durch nonverbale akustische Signale mit einer drahtlosen Schnittstelle zur Kommunikation mit einer externen Sprachausgabeeinheit wird es einem Benutzer durch die vorliegende Erfindung ermöglicht, bei der überwiegenden Zahl der durchzuführenden Messungen den Komfort einer Sprachausgabe zu nutzen, ohne daß eine Sprachausgabeeinheit mitgeführt werden muß. Befindet sich der Benutzer bei Messungen in seiner Wohnung, so kann eine Sprachausgabe über die drahtlose Schnittstelle und die Sprachausgabeeinheit erfolgen. Für Situationen, in denen der Benutzer Messungen außerhalb seiner Wohnung vornimmt, steht eine in das Analysehandgerät integrierte akustische Signalausgabeeinrichtung zur Verfügung, mit der Meßergebnisse durch nonverbale akustische Signale ausgegeben werden können. Selbst für einen blinden Benutzer entfällt deshalb die Notwendigkeit, die Sprachausgabeeinheit ständig mit sich herumzutragen.

Meßergebnisse und andere Informationen können von der akustischen Signalausgabeeinrichtung beispielsweise durch Systeme Morse-ähnlicher Pieptöne oder Pieptöne verschiedener Frequenz, die sprach- und kulturkreisunabhängig leicht verständlich und leicht erlernbar sind, ausgegeben werden.

Die drahtlose Schnittstelle eines erfindungsgemäßen Analysehandgeräts enthält einen Sender und einen Empfänger. In entsprechender Weise enthält auch die Schnittstelle der externen Sprachausgabeeinheit einen Sender und einen Empfänger. Bevorzugt sind der Sender und der Empfänger des Analysehandgeräts ebenso wie der Sender und der Empfänger der externen Sprachausgabeeinheit jeweils zu einer Sender- und Empfängereinheit zusammengefaßt.

Ein weiterer Vorteil der vorliegenden Erfindung liegt darin, daß sehbehinderte Benutzer die Sprachausgabeeinheit für mehrere Generationen von Analysehandgeräten verwenden können. Die Verwendung eines genormten Kommunikationsprotokolls und einer im wesentlichen gleichbleibenden drahtlosen Schnittstelle ermöglicht die Kompatibilität unterschiedlicher Analysehandgeräte mit derselben Sprachausgabeeinheit. Durch die Trennung von Analysegerät und Sprachausgabeeinheit kann in idealer Weise auf die speziellen Anforderungen sehbehinderter Benutzer eingegangen werden und unter Ausnutzung der Kostenvorteile einer Serienfertigung ein komfortables und preiswertes Analysesystem zur Verfügung gestellt werden.

Die Erfindung wird nachfolgend anhand eines in den beigefügten Figuren dargestellten Ausführungsbeispiels näher erläutert. Die darin dargestellten Besonderheiten können einzeln oder in Kombination verwendet werden, um bevorzugte Ausgestaltungen zu schaffen. Es zeigen:
- Fig. 1: ein Ausführungsbeispiel eines erfindungsgemäßen Analysehandgeräts, und
- Fig. 2: ein Ausführungsbeispiel einer externen Sprachausgabeeinheit für das in Figur 1 gezeigte Analysehandgerät.

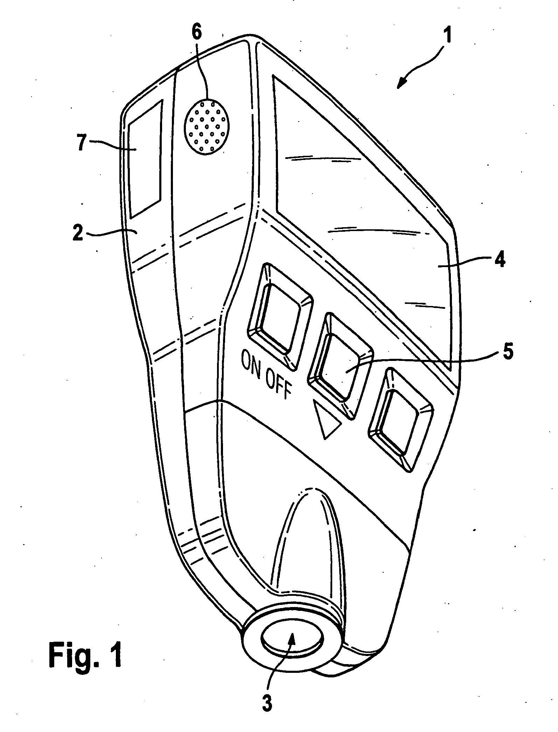

Bei dem in Figur 1 gezeigten Analysehandgerät 1 handelt es sich um ein Gerät zur Messung des Blutglucosegehalts für Diabetiker. In dem Gerät befindet sich eine Lanzette (nicht dargestellt), mit der in einem Körperteil, beispielsweise einem Finger, der an einer Öffnung 3 des Gehäuses 2 angelegt wird, eine Einstichwunde erzeugt werden kann. Das Gerät 1 nimmt selbsttätig aus der Einstichwunde austretendes Blut auf und ermittelt mit einer internen Meßeinrichtung (nicht dargestellt) einen Blutglucosekonzentrationswert. Zum Betätigen des Geräts 1 und/oder zum Benutzen von Sonderfunktionen sind Bedienungselemente 5 in Form von Tasten vorhanden.

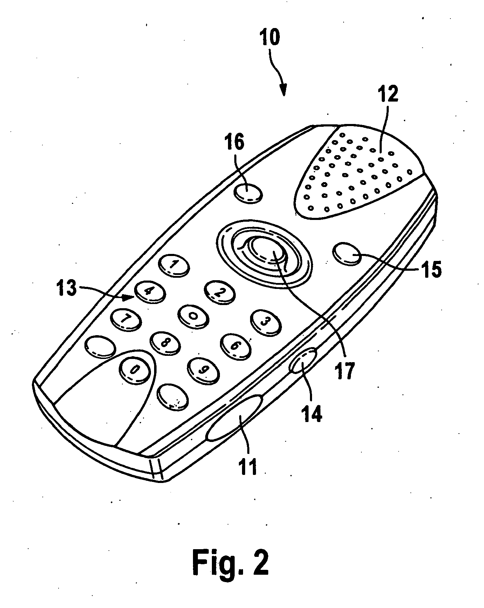

Das Analysehandgerät 1 weist eine Ausgabeeinrichtung zum Ausgeben von Meßergebnissen, die mittels der Meßeinrichtung ermittelt wurden, auf. Meßeinrichtung und Ausgabeeinrichtung sind an eine netzunabhängige Stromquelle, beispielsweise Batterien oder Solarzellen, angeschlossen. Die Ausgabeeinrichtung umfaßt sowohl eine akustische Signalausgabeeinrichtung 6 zur Ausgabe der Meßergebnisse durch nonverbale akustische Signale, beispielsweise Pieptöne, als auch eine drahtlose Schnittstelle 7 zur Kommunikation mit einer externen Sprachausgabeeinheit (Figur 2), über die Meßergebnisse verbal ausgegeben werden können.

Bei der drahtlosen Schnittstelle 7 handelt es sich um eine Infrarotschnittstelle, in die ein Infrarotsender und ein Infrarotempfänger integriert sind, so daß durch Infrarotstrahlung Daten mit der externen Sprachausgabeeinheit ausgetauscht werden können. Anstelle einer Infrarotschnittstelle mit einem Infrarotsender und Infrarotempfänger kann auch eine Datenübertragung per Funk oder durch Ultraschall zur Kommunikation zwischen dem Analysehandgerät 1 und der externen Sprachausgabeeinheit eingesetzt werden.

Die Ausgabeeinrichtung hat einen Akustikmodus, in dem Meßergebnisse mittels der akustischen Signalausgabeeinrichtung 6 ausgegeben werden, und einen Sprachausgabemodus, in dem Meßergebnisse über die Infrarotschnittstelle 7 und die externe Sprachausgabeeinheit ausgegeben werden. Ein Übergang der Ausgabeeinrichtung aus dem Akustikmodus in den Sprachausgabemodus wird durch Empfang eines von der Sprachausgabeeinheit ausgesandten Betriebsbereitschaftssignals ausgelöst.

Die Ausgabeeinrichtung des in Figur 1 dargestellten Analysehandgeräts 1 umfaßt zusätzlich eine optische Anzeigeenrichtung 4 in Form eines Displays. Durch ein von dem Benutzer betätigbares Bedienungselement 5 kann die Ausgabeeinrichtung zwischen dem Akustikmodus, in dem Meßergebnisse mittels der akustischen Signalausgabeeinrichtung 6 ausgegeben werden, und einem Optikmodus, in dem Meßergebnisse mittels der optischen Anzeigeeinrichtung 4 ausgegeben.werden, umgeschaltet werden. Bevorzugt werden in dem Akustikmodus Meßergebnisse zusätzlich mit der Anzeigeeinrichtung 4 angezeigt. Ebenso werden bevorzugt auch in dem Sprachausgabemodus Meßergebnisse zusätzlich mit der Anzeigeeinrichtung 4 angezeigt.

Selbstverständlich können die akustische Signalausgabeeinrichtung 6, die externe Sprachausgabeeinheit und die optische Anzeigeeinrichtung 4 nicht nur zur Ausgabe von Meßergebnissen, sondern zusätzlich auch zur Ausgabe von Bedienungsinformationen, insbesondere über den Status des Geräts 1, genutzt werden. Bevorzugt erfolgt eine Ausgabe von Bedienungsinformationen auch in dem Sprachausgabemodus zusätzlich über die akustische Signalausgabeeinrichtung und die Anzeigeeinrichtung 4. Beispielsweise kann ein Gerätezustand durch eine Pieptonsequenz angezeigt werden, beispielsweise Ergebnisse von Selbsttests des Analysehandgeräts oder der Ladezustand der Batterien. Ein wichtiger Gerätezustand in diesem Sinne betrifft ferner die Frage, ob das Gerät 1 für einen Einstich und eine anschließende Blutglucosekonzentrationsmessung bereit ist oder ob eine Probe korrekt aus einen an der Öffnung 3 positionierten Figur entnommen wurde. Eine Ausgabe von derartigen Bedienungsinformationen über die akustische Signalausgabeeinrichtung 6 ist insbesondere auch deshalb vorteilhaft, weil beim Haritieren mit dem Gerät 1 die Infrarotschnittstelle 7 zeitweise verdeckt oder durch Sonnenlicht gestört werden kann, so daß kein Datenaustausch mit der externen Sprachausgabeeinheit mehr möglich ist.

In Figur 2 ist die externe Sprachausgabeeinheit 10 für das anhand von Figur 1 beschriebene Analysehandgerät 1 dargestellt. Die Sprachausgabeeinheit 10 hat eine drahtlose Schnittstelle 11 zur Kommunikation mit dem Analysehandgerät 1 über dessen drahtlose Schnittstelle 7. Die Schnittstelle 11 der Sprachausgabeeinheit 10 ist ebenso wie die Schnittstelle 7 des Analysehandgeräts 1 für einen bidirektionalen Informationsaustausch ausgebildet. Im Falle von Infrarotschnittstellen haben die beiden Schnittstellen 7 und 11 deshalb einen Infrarotsender und einen Infrarotempfänger.

Meßergebnisse und/oder Bedienungsinformationen können von der Sprachausgabeeinheit 10 verbal über einen Lautsprecher 12 ausgegeben werden. Die Sprachausgabeeinheit 10 weist ferner Bedienungselemente in Form einer Tastatur 13, einer seitlich angebrachten Taste 14 zur Regelung der Lautstärke, zusätzlichen Bedienungstasten 15,16 und einem Navigationskreuz 17 auf.

Das Navigationskreuz 17 entspricht in seinem Aufbau und seiner Funktion den für Laptops gebräuchlichen Navigationskreuzen, mit denen ein Cursor auf einem Bildschirm bewegt werden kann, beispielsweise um Punkte aus einem komplexen Menü auszuwählen. Bei der dargestellten Sprachausgabeeinheit 10 ist ein derartiger Bildschirm nicht vorhanden, da er für blinde Benutzer ohnehin nicht nutzbar wäre. Ein blinder Benutzer kann jedoch mit dem Navigationskreuz 17 Einträge aus einer virtuellen Tabelle auswählen. Bei diesen Einträgen kann es sich beispielsweise um Unterpunkte eines Funktionsmenüs oder um historische Meßdaten handeln, die in einer Meßwertetabelle gespeichert sind.

Die Speicherung von Meßdaten ist für sehbehinderte Benutzer von großer Bedeutung, da diese Personen nicht oder nur mit großer Mühe für einen behandelnden Arzt medizinische Daten in einem Tagebuch festhalten können. Durch einen Speicher (nicht gezeigt) der externen Analyseeinheit 10 wird Benutzern deshalb eine Tagebuchfunktion ermöglicht. Diese Tagebuchfunktion wird durch Eingabemöglichkeiten über die numerische Tastatur 13 und das Navigationskreuz 17 unterstützt.

Während das Analysehandgerät 1 über eine netzunabhängige Stromquelle, beispielsweise Batterien, verfügt, kann die Sprachausgabeeinheit 10 mit einem Stromkabel zum Anschluß an das allgemeine Stromnetz ausgestattet werden. Bevorzugt ist jedoch auch die Sprachausgabeeinheit 10 mit einer netzunabhängigen Stromquelle, bevorzugt Batterien, ausgestattet.

Das Analysehandgerät 1 und die externe Sprachausgabeeinheit 10 bilden ein Analysesystem, das für sehbehinderte Personen einen hohen Benutzungskomfort bietet. Die Sprachausgabeeinheit 10 sendet in vorgegebenen Zeitabständen von etwa einer halben Minute Betriebsbereitsschaftssignale aus. Empfängt das Analysehandgerät 1 ein solches Betriebsbereitsschaftssignal, so antwortet es mit einem Identifikationssignal und tauscht mit der Sprachausgabeeinheit 10 eine Reihe von Synchronisationsimpulsen gemäß einem Kommunikationsprotokoll aus, wobei auch ein Abgleich von Datum und Uhrzeit erfolgt. Bei der Kommunikation übernimmt die Sprachausgabeeinheit 10 eine Masterfunktion und erfragt Betriebs- und Gerätedaten des Analysehandgeräts 1. Auf diese Weise werden der Sprachausgabeeinheit 10 beispielsweise Gerätetyp und Seriennummer des Analysehandgeräts 1, der Gerätestatus und dessen Speicherinhalt mitgeteilt. Nachdem sich das Analysehandgerät 1 und die Sprachausgabeeinheit 10 über das Kommunikationsprotokoll verständigt haben, wechselt die Ausgabeeinrichtung des Analysehandgeräts 1 aus dem Akustikmodus in den Sprachausgabemodus und überträgt die auszugebenden Meßergebnisse. Durch Drücken der Repeat-Taste 15 der Sprachausgabeeinheit 10 kann eine Wiederholung einer Ansage von Meßergebnissen veranlaßt werden

Der Kommunikationsausbau zwischen dem Analysehandgerät 1 und der externen Sprachausgabeeinheit 10 kann prinzipiell von jedem der beiden Geräte initiiert werden. Es ist deshalb auch möglich, daß das Analysehandgerät 1 nach jedem Einschalten ein Suchsignal aussendet und die Sprachausgabeeinheit 10 darauf antwortet. Für beide Fälle des Kommunikationsaufbaus ist es bevorzugt, daß die Sprachausgabeeinheit 10 eine Masterfunktion übernimmt und Betriebs- und Gerätedaten des Analysehandgeräts 1 erfragt. Die Sprachausgabeeinheit 10 vergleicht dabei ein von dem Analysehandgerät 1 ausgesandtes Identifikationssignal mit einem gespeicherten Identifikationssignal. Falls dieser Vergleich keine Übereinstimmung zeigt, wird die Kommunikation abgebrochen, so daß nur Meßdaten des Benutzers von der Sprachausgabeeinheit ausgegeben und gespeichert werden. Für einen raschen Kommunikationsaufbau ist es günstig, wenn die Schnittstelle 7 des Analysehandgeräts 1 gleichzeitig senden und empfangen kann. Dies kann mit einer entsprechenden Steuereinheit, beispielsweise in Form eines geeignet programmierten Mikroprozessors, erreicht werden. Möglich ist es auch, daß sowohl das Analysehandgerät 1 als auch die externe Sprachausgabeeinheit 10 periodisch nach dem Senden von Daten auf Empfang geschaltet werden. Bevorzugt werden dabei für Analysehandgeräte 1 und die Sprachausgabeinheit 10 unterschiedliche Periodendauern verwendet.

Wird die Kommunikation zwischen Analysehandgerät 1 und der externen Sprachausgabeeinheit 10 unterbrochen oder mißlingt der Kommunikationsaufbau der beiden Geräte 1, 10 über das vorgegebene Kommunikationsprotokoll, so wechselt das Analysehandgerät 1 automatisch in den Akustikmodus. Bei der Kommunikation tauschen die Ausgabeeinrichtung und die Sprachausgabeeinheit 10 im Rahmen des Kommunikationsprotokolls regelmäßig Bestätigungssignale aus. Bei Ausbleiben eines Bestätigungssignals der Sprachausgabeeinheit 10 wechselt die Ausgabeeinrichtung selbsttätig in den Akustikmodus.

Ein wichtiger Vorteil der externen Sprachausgabeeinheit 10 besteht darin, daß einem Benutzer nicht nur Meßergebnisse verbal mitgeteilt werden können, sondern auch Status- und Bedienungsinformationen des Analysehandgeräts 1 detailliert und ausführlich zur Verfügung gestellt werden können. Die Sprachausgabeeinheit 10 verfügt zu diesem Zweck über einen Speicher (nicht dargestellt), in dem das Benutzerhandbuch des Analysesystems auszugsweise oder vollständig, bevorzugt in Form von MP3 Dateien, gespeichert ist, so daß dessen Inhalt einem Benutzer verbal zugänglich gemacht werden kann.

Durch Drücken der Hilfe-Taste 16 wird eine Hilfefunktion aktiviert und eine verbale Ausgabe der für den aktuell vorhandenen Betriebs- oder Bedienungszustand des Analysesystems relevanten Teile des Benutzerhandbuchs veranlaßt. Wird die Hilfe-Taste 16 während einer verbalen Ausgabe von Hilfe-Informationen erneut betätigt, so wird die Ausgabe unterbrochen und erst bei nochmaliger Betätigung der Taste 16 fortgesetzt. Auf diese Weise hat der Benutzer die Möglichkeit, die Geschwindigkeit des Informationsflusses an seine Aufnahmefähigkeit anzupassen.

Bei dem beschriebenen Analysesystem kann ein Benutzer über die Bedienungselemente 5 des Analysehandgeräts zwischen drei verschiedenen Betriebsarten der akustischen Signalausgabeeinrichtung 6 wählen. Die erste Betriebsart ist der beschriebene Akustikmodus, in dem sowohl Meßergebnisse als auch Statusinformationen des Geräts 1 durch Pieptöne ausgegeben werden. Die zweite Betriebsart ist ein eingeschränkter Akustikmodus, in dem lediglich Statusinformationen durch Pieptöne ausgegeben werden und eine Ausgabe von Meßergebnissen nur über die Sprachausgabeeinheit 10 und/oder die optische Anzeigeeinrichtung 4 erfolgt. In einer dritten Betriebsart kann die akustische Signalausgabeeinrichtung 6 ganz abgeschaltet werden (Optikmodus, Stummschaltung), so daß weder Meßergebnisse noch Statusinformationen durch Pieptöne ausgegeben werden. In den letztgenannten Betriebsarten kann das Analysehandgerät 1 auch von normalsichtigen Personen benutzt werden, die sich möglicherweise durch Pieptöne gestört fühlen würden. Auf diese Weise kann das Analysehandgerät 1 in größeren Stückzahlen produziert werden, so daß sich durch eine entsprechende Serienfertigung Kostenvorteile ergeben.

## Patentansprüche

1. Analysehandgerät zur Untersuchung einer medizinisch bedeutsamen Probe, insbesondere zur Ermittlung der Blutglucosekonzentration von Diabetikern, umfassend
eine Meßeinrichtung zum Messen der Konzentration eines Analyten in der Probe,
eine Ausgabeeinrichtung (4,6,7) zum Ausgeben von Meßergebnissen, die mittels der Meßeinrichtung ermittelt wurden,
**dadurch gekennzeichnet, dass**
die Ausgabeeinrichtung (4,6,7) sowohl eine akustische Signalausgabeeinrichtung (6) zur Ausgabe der Meßergebnisse durch nonverbale akustische Signale als auch eine drahtlose Schnittstelle (7) zur Kommunikation mit einer externen Sprachausgabeeinheit (10) umfaßt, über die Meßergebnisse verbal ausgegeben werden können,
die Ausgabeeinrichtung (4,6,7) einen Akustikmodus, in dem Meßergebnisse mittels der akustischen Signalausgabeeinrichtung (6) ausgegeben werden, und einen Sprachausgabemodus, in dem Meßergebnisse über die Schnittstelle (7) ausgegeben werden, hat, und
ein Übergang der Ausgabeeinrichtung (4,6,7) aus dem Akustikmodus in den Sprachausgabemodus durch Empfang eines von der Sprachausgabeeinheit (10) ausgesandten Signals ausgelöst wird.

2. Analysehandgerät nach Anspruch 1, **dadurch gekennzeichnet, daß** die akustische Signalausgabeeinrichtung (6) zum Ausgeben der Meßergebnisse Pieptöne erzeugt.

3. Analysehandgerät nach Anspruch 1, **dadurch gekennzeichnet, daß** die Ausgabeeinrichtung (4,6,7) bei Ausbleiben eines Bestätigungssignals der Sprachausgabeeinheit (10) selbsttätig aus dem Sprachausgabemodus in den Akustikmodus zurückkehrt.

4. Analysehandgerät nach einem der vorhergehenden Ansprüche, **dadurch gekennzeichnet, daß** die Ausgabeeinrichtung (4,6,7) zusätzlich eine optische Anzeigeeinrichtung (4) aufweist.

5. Analysehandgerät nach Anspruch 4, **dadurch gekennzeichnet, daß** es ein von dem Benutzer betätigbares Bedienungselement (5) aufweist, mit dem die Ausgabeeinrichtung (4,6,7) zwischen einem Akustikmodus, in dem Meßergebnisse mittels der akustischen Signalausgabeeinrichtung (6) ausgegeben werden, und einem Optikmodus, in dem Meßergebnisse mittels der optischen Anzeigeeinrichtung (4) ausgegeben werden, umschaltbar ist.

6. Analysehandgerät nach Anspruch 4, **dadurch gekennzeichnet, daß** in dem Akustikmodus und/oder dem Sprachausgabemodus Meßergebnisse zusätzlich mit der optischen Anzeigeeinrichtung (4) angezeigt werden

7. Analysehandgerät nach einem der vorhergehenden Ansprüche, **dadurch gekennzeichnet, daß** die Schnittstelle (7) eine Infrarotschnittstelle ist.

8. Analysehandgerät nach einem der vorhergehenden Ansprüche, **dadurch gekennzeichnet, daß** in dem Sprachausgabemodus eine Ausgabe von Bedienungsinformationen über die akustische Signalausgaberichtung (6) und/oder die Anzeigeeinrichtung (4) erfolgt.

9. Analysesystem umfassend ein Analysehandgerät (1) nach einem der vorhergehenden Ansprüche und eine Sprachausgabeeinheit (10), die eine drahtlose Schnittstelle (11) zur Kommunikation mit dem Analysehandgerät (1) aufweist.

10. Sprachausgabeeinheit mit einer drahtlosen Schnittstelle (11) zur Kommunikation mit einem Analysehandgerät (1) nach einem der Ansprüche 1 bis 8.

## Claims

1. Handheld analysis instrument for assaying a medically significant sample, in particular for determining the blood glucose concentration of diabetics, comprising
a measuring device for measuring the concentration of an analyte in the sample,
an output device (4, 6, 7) for outputting measurement results determined by the measuring device,
**characterized in that**
the output device (4, 6, 7) comprises an acoustic signal output device (6) for outputting the measurement results through nonverbal acoustic signals and a wireless interface (7) for communicating with an external speech output unit (10), by which the measurement results may be outputted verbally,
the output device (4, 6, 7) operates in an acoustic mode, in which measurement results are outputted using the acoustic signal output device (6), and in a speech output mode, in which measurement results are outputted via the interface (7), and
a transition of the output device (4, 6, 7) from the acoustic mode into the speech output mode is triggered by receiving a signal transmitted by the speech output unit (10).

2. Handheld analysis instrument according to claim 1, **characterized in that** the acoustic signal output device (6) generates beeps for outputting the measurement results.

3. Handheld analysis instrument according to claim 1, **characterized in that** the output device (4, 6, 7) returns automatically from the speech output mode into the acoustic mode if it does not receive a confirmation signal of the speech output unit (10).

4. Handheld analysis instrument according to any one of the preceding claims, wherein the output device (4, 6, 7) additionally comprises an optical display device (4).

5. Handheld analysis instrument according to claim 4, **characterized in that** it has an operating element (5), which may be actuated by the user and by which the output device (4, 6, 7) may be switched over between an acoustic mode, in which measurement results are outputted using the acoustic signal output device (6), and a visual mode, in which measurement results are outputted using the optical display device (4).

6. Handheld analysis instrument according to claim 4, **characterized in that** in the acoustic mode and/or the speech output mode measurement results are additionally displayed using the optical display device (4).

7. Handheld analysis instrument according to any one of the preceding claims, **characterized in that** the interface (7) is an infrared interface.

8. Handheld analysis instrument according to any one of the preceding claims, wherein, in the speech output mode, operating information is outputted via the acoustic signal output device (6) and/or the display device (4).

9. An analysis system comprising a handheld analysis instrument (1) according to any one of the preceding claims and a speech output unit (10), which has a wireless interface (11) for communicating with the handheld analysis instrument (1).

10. Speech output unit having a wireless interface (11) adapted for communication with a handheld analysis instrument (1) according to any one of claims 1 through 8.

## Revendications

1. Appareil d'analyse portable pour examiner un prélèvement médicalement significatif, en particulier pour déterminer la concentration de glucose dans le sang de personnes diabétiques, comprenant
■ un dispositif de mesure destiné à mesurer la concentration d'une substance à analyser dans le prélèvement,
■ un dispositif de sortie (4, 6, 7) destiné à délivrer en sortie des résultats de mesure qui ont été déterminés au moyen du dispositif de mesure,
**caractérisé en ce que**
■ le dispositif de sortie (4, 6, 7) comporte un dispositif d'émission de signal acoustique (6) destiné à délivrer en sortie les résultats de mesure par des signaux acoustiques non verbaux ainsi qu'une interface sans fil (7) à des fins de communication avec une unité de sortie vocale externe (10) par le biais de laquelle les résultats de mesure peuvent être émis verbalement,
■ le dispositif de sortie (4, 6, 7) a un mode acoustique, dans lequel des résultats de mesure sont délivrés en sortie au moyen du dispositif d'émission de signal acoustique (6), et un mode de sortie vocale, dans lequel des résultats de mesure sont délivrés en sortie par le biais de l'interface (7), et
■ une transition du dispositif de sortie (4, 6, 7) du mode acoustique dans le mode de sortie vocale est déclenchée par la réception d'un signal émis par l'unité de sortie vocale (10).

2. Appareil d'analyse portable selon la revendication 1, **caractérisé en ce que** le dispositif d'émission de signal acoustique (6), destiné à délivrer en sortie les résultats de mesure, génère des tonalités bip.

3. Appareil d'analyse portable selon la revendication 1, **caractérisé en ce que**, en absence de signal de confirmation de l'unité de sortie vocale (10), le dispositif de sortie (4, 6, 7) retourne automatiquement du mode de sortie vocale dans le mode acoustique.

4. Appareil d'analyse portable selon l'une quelconque des revendications précédentes, **caractérisé en ce que** le dispositif de sortie (4, 6, 7) comporte en plus un dispositif d'affichage optique (4).

5. Appareil d'analyse portable selon la revendication 4, **caractérisé en ce qu'**il comporte un élément de commande (5), actionnable par l'utilisateur, avec lequel le dispositif de sortie (4, 6, 7) peut être commuté entre un mode acoustique, dans lequel des résultats de mesure sont délivrés en sortie au moyen du dispositif d'émission de signal acoustique (6), et un mode optique, dans lequel des résultats de mesure sont délivrés en sortie au moyen du dispositif d'affichage optique (4).

6. Appareil d'analyse portable selon la revendication 4, **caractérisé en ce que** des résultats de mesure sont affichés en plus avec le dispositif d'affichage optique (4) dans le mode acoustique et/ou le mode de sortie vocale.

7. Appareil d'analyse portable selon l'une quelconque des revendications précédentes, **caractérisé en ce que** l'interface (7) est une interface infrarouge.

8. Appareil d'analyse portable selon l'une quelconque des revendications précédentes, **caractérisé en ce que**, dans le mode de sortie vocale, les informations de commande sont délivrées en sortie par le biais du dispositif d'émission de signal acoustique (6) et/ou le dispositif d'affichage (4).

9. Système d'analyse comportant un appareil d'analyse portable (1) selon l'une quelconque des revendications précédentes et une unité de sortie vocale (10), qui comporte une interface sans fil (11) à des fins de communication avec l'appareil d'analyse portable (1).

10. Unité de sortie vocale avec une interface sans fil (11) à des fins de communication avec un appareil d'analyse portable (1) selon l'une quelconque des revendications 1 à 8.
